**Europäisches Patentamt**

(19) **European Patent Office**

**Office européen des brevets**

(11) Veröffentlichungsnummer: **0 067 394**
**B1**

(12) **EUROPÄISCHE PATENTSCHRIFT**

(45) Veröffentlichungstag der Patentschrift:
**20.03.85**

(51) Int. Cl.⁴: **C 12 Q 1/60,** G 01 N 33/92

(21) Anmeldenummer: **82104965.7**

(22) Anmeldetag: **07.06.82**

(54) **Wässrige Cholesterin-Standardlösung und Verfahren zu ihrer Herstellung.**

(30) Priorität: **10.06.81 DE 3122917**

(43) Veröffentlichungstag der Anmeldung:
**22.12.82 Patentblatt 82/51**

(45) Bekanntmachung des Hinweises auf die Patenterteilung:
**20.03.85 Patentblatt 85/12**

(84) Benannte Vertragsstaaten:
**AT BE CH DE FR GB IT LI LU NL SE**

(56) Entgegenhaltungen:
**DE - A - 2 839 433**
**DE - C - 2 324 386**
**US - A - 4 189 400**

**CLINICAL CHEMISTRY, Band 25, Nr. 1, 1979 Easton J. ABELE et al. "Aqueous Primary Standard for Use in Measuring Cholesterol by the Cholesterol Oxidase Method" Seiten 132 bis 135**

(73) Patentinhaber: **Boehringer Mannheim GmbH, Sandhoferstrasse 116, D-6800 Mannheim 31 (DE)**

(72) Erfinder: **Deeg, Rolf, Dr. rer. nat., Seeseitener Strasse 18, D-8124 Seeshaupt (DE)**
Erfinder: **Dengler, Gisela, Alpspitzstrasse 1, D-8121 Wielenbach (DE)**
Erfinder: **Ziegenhorn, Joachim, Dr. rer. nat., Ina-Seidel-Weg 1, D-8130 Starnberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Cholesterin-Standard in Form einer wässrigen Cholesterin-Lösung, die neben einem bestimmten und stabilen Gehalt an Cholesterin ein Detergensgemisch aus Cholsäure und Desoxycholsäure bzw. aus geeigneten Salzen oder Derivaten dieser Säuren enthält.

Es ist eine Reihe von chemischen und enzymatischen Verfahren zur quantitativen Bestimmung des Cholesterins in verschiedenen Materialien, insbesondere in biologischen Flüssigkeiten, bekannt bzw. vorgeschlagen worden. Bei diesen Verfahren ist es fast stets erforderlich, zur Auswertung der Messergebnisse Cholesterin-Lösungen mit bestimmtem, bekanntem Gehalt an Cholesterin als Standard in die Messung einzubeziehen. Man verwendet zu diesem Zweck Cholesterin-Lösungen mit definiertem Cholesteringehalt, die als Cholesterin-Standard bezeichnet werden.

Für die Bestimmung des Cholesterins in biologischen Flüssigkeiten sind in der Regel Standardlösungen erforderlich, deren Eigenschaften mit denen der biologischen Flüssigkeit weitgehend übereinstimmen. Diese Forderung lässt sich im allgemeinen durch die Verwendung von wässrigen Cholesterin-Standardlösungen erfüllen. Bei einer Cholesterin-Standardlösung kommt es ferner darauf dan, dass über einen längeren Zeitraum hinweg die Lösung ausreichend stabil ist und der Cholesteringehalt absolut konstant bleibt. Es sind bisher eine Reihe von wässrigen Cholesterin-Standardlösungen bekanntgeworden, die einen konstanten, über einen längeren Zeitraum stabilen Cholesteringehalt aufweisen. So wird beispielsweise in dem deutschen Patent 2 324 386 eine wässrige Cholesterin-Standardlösung beschrieben und beansprucht, die neben einem Lösungsvermittler, beispielsweise Oxypolyethoxydodecan, und anderen Inhaltsstoffen einen Gehalt von 1-20 Volumenprozent eines primären oder sekundären aliphatischen Alkohols mit 1-4 Kohlenstoffatomen aufweist. Diese Cholesterin-Standardlösung hat sich als stabil erwiesen und zeigt selbst bei jahrelanger Lagerung unter normalen Bedingungen einen unveränderten Cholesteringehalt.

Aus der DE-OS 2 839 433 ist eine wässrige Lipid-Standardlösung bekannt, die neben einem Lipid ein ionisches unnd ein nicht-ionisches Detergens enthält. Mit dieser Zusammensetzung soll vor allem erreicht werden, dass beim unvermeidlichen Stehen der Proben möglichst wenig verdunstet und die Viskosität der Standardlösung nicht beeinflusst wird.

Aus Clin. Chem. <u>25</u> (1979), Seite 132-135 schliesslich ist ein wässriger Cholesterin-Standard bekannt, der neben einer bestimmten und konstanten Menge Cholesterin Natriumdesoxycholat als Löslichkeitsvermittler aufweist und der sich für die enzymatische Bestimmung von Cholesterin als nützlich erwiesen hat.

Die bisher bekannten Cholesterin-Standardlösungen sind zwar mehr oder weniger brauchbar für enzymatische Cholesterinbestimmungen nach den sogenannten Endwert-Methoden; für eine kinetische Cholesterinbestimmung, wie sie beispielsweise in der deutschen Patentanmeldung P 30 46 241.0 beschrieben worden ist, sind sie jedoch nicht verwendbar. Hierfür ist ein Cholesterin-Standard erforderlich, der neben den bisherigen Erfordernissen an einen solchen Standard hinsichtlich Stabilität, Viskosität noch das zusätzliche Erfordernis erfüllen muss, dass sein kinetisches Verhalten identisch ist mit demjenigen der zu bestimmenden Probe. Dis bisher bekanntgewordenen Cholesterin-Standardlösungen sind für eine kinetische Cholesterinbestimmung nicht brauchbar, da ihr kinetisches Verhalten aufgrund ihres Detergensgehaltes deutlich von demjenigen der zu messenden Cholesterinproben abweicht.

Aufgabe der vorliegenden Erfindung war es daher, eine alkoholfreie, stabile, wässrige Cholesterin-Standardlösung zur Verfügung zu stellen, die in ihrem kinetischen Verhalten bei der enzymatischen Cholesterinbestimmung demjenigen der zu messenden Cholesterinproben gleicht.

Gelöst wird diese Aufgabe erfindungsgemäss durch eine wässrige Cholesterin-Standardlösung, die neben einem bestimmten Gehalt an Cholesterin ein Detergensgemisch aus 10-90% Cholsäure und 90-10% Desoxycholsäure bzw. aus geeigneten Salzen oder Derivaten dieser Säuren enthält.

Gegenstand der Erfindung ist daher eine wässrige Cholesterin-Standardlösung mit bestimmtem Gehalt an Cholesterin, dadurch gekennzeichnet, dass es ein Detergensgemisch aus 10-90% Cholsäure und 90 bis 10% Desoxycholsäure bzw. aus geeigneten Salzen oder Derivaten dieser Säuren enthält.

Eine Lösung von Cholesterin in dem beanspruchten Detergensgemisch aus Cholsäure und Desoxycholsäure bzw. aus geeigneten Salzen oder Derivaten dieser Säuren hat sich als über längere Zeit stabil und von hinreichend geringer Viskcsität erwiesen. Es hat sich darüber hinaus gezeigt, dass durch Kombination der beiden Detergentien überraschenderweise das kinetische Verhalten des erfindungsgemässen Cholesterin-Standards optimal dem kinetischen Verhalten der cholesterinhaltigen Probe angepasst werden kann.

Bevorzugt wird im Rahmen der erfindungsgemässen Cholesterin-Standardlösung ein Detergensgemisch, das die Cholsäure und Desoxycholsäure bzw. deren Salze oder geeignete Derivate in einem Verhältnis von 40:60 - 60:40 enthält. Für eine kinetische Cholesterinbestimmung gemäss der deutschen Patentanmeldung P 30 46 241.0 hat sich besonders vorteilhaft ein Cholesterin-Standard erwiesen, der als Detergensgemisch gleiche Anteile an Natriumcholat und Natriumdesoxycholat aufweist. Die erfindungsgemässe Cholesterin-Standardlösung enthält 10-300 g/l vorzugsweise 50-100 g/l des Detergensgemisches.

Gemäss einer weiteren bevorzugten Ausführungsform enthält die erfindungsgemässe Cholesterin-Standardlösung zusätzlich einen Puffer, dessen Pufferbereich bei einem pH-Wert von 7-9 liegt. Die Pufferkonzentration bewegt sich vorzugsweise in dem Bereich von 10-100 mmol/l. Besonders vorteilhaft hat sich die Verwendung von Tris-Puffer, pH 8,0 $\pm$ 0,2 in einer Konzentration von 50 mmol/l erwiesen.

Es hat sich darüber hinaus als zweckmässig herausgestellt, zur Verhinderung eines Bakterienbefalles ein oder mehrere geeignete Konservierungsmittel zuzusetzen. Geeignet sind alle Konservierungsmittel, die das kinetische Verhalten des Cholesterin-Standards nicht beeinflussen, beispielsweise Azid, Germal oder Chloracetamid. Das oder die Konservierungsmittel werden der Standardlösung in einer Konzentration von 0,1-10 mg/ml zugesetzt.

Die Cholsäure bzw. Desoxycholsäure wird entweder als solche oder vorzugsweise in Form ihrer Salze, wobei die Natriumsalze besonders bevorzugt sind, oder in Form von Derivaten eingesetzt. Als Derivate kommen beispielsweise in Frage die Glyko- oder Tauro-Cholsäure bzw. die Glyko- oder Tauro-Desoxycholsäure.

Ein im Sinne der vorliegenden Erfindung besonders bevorzugter Cholesterin-Standard enthält:

| 50-400 mg/dl | Cholesterin |
| 50 g/l | Natriumcholat |
| 50 g/l | Natriumdesoxycholat |
| 1,0 g/l | Natriumazid |
| 2,5 g/l | Chloracetamid |
| 50 mmol/l | Tris-Puffer, pH 8,0 ± 0,2 |

Zur Herstellung der erfindungsgemässen Cholesterin-Standardlösung wird zunächst die Cholsäure und Desoxycholsäure bzw. deren geeignete Salze oder Derivate in destilliertem Wasser oder 0,9%iger Kochsalzlösung, die zusätzlich Konservierungsmittel und einen Puffer enthalten kann, aufgelöst und danach die bestimmte Menge Cholesterin (bis zu 400 mg/100 ml Lösungsmittelgemisch) unter Rühren und leichtem Erwärmen, vorzugsweise auf 40-60°C eingebracht.

Die Auflösung des Cholesterins in der detergenshaltigen Lösung kann auch vorteilhaft durch Ultraschallbehandlung erfolgen. Das Herstellungsverfahren lässt sich ferner derart modifizieren, dass man die Detergentien und das Cholesterin in einem geeigneten, leicht flüchtigen organischem Lösungsmittel, beispielsweise Methanol, auflöst, die erhaltene Lösung eindampft und den Rückstand wieder in destilliertem Wasser oder 0,9%iger Kochsalzlösung, die ein Konservierungsmittel oder einen Puffer enthalten kann, aufnimmt.

Die erfindungsgemässe Cholesterin-Standardlösung ist jedoch nicht nur bei der kinetischen Cholesterinbestimmung einsetzbar. Sie lässt sich in gleicher Weise bei der üblichen enzymatischen Endwert-Methode einsetzen. Sie ist ferner geeignet zur Bestimmung von HDL-Cholesterin. Hierzu kann der Standard vorteilhafterweise verdünnt werden. Dies wird zweckmässig mit destilliertem Wasser oder dem für die Probenvorbehandlung verwendeten Fällungsreagens, beispielsweise Phosphorwolframsäure/Magnesiumionen, durchgeführt.

Die Erfindung wird durch die folgenden Beispiele näher erläutert:

*Beispiel 1*

50 g Natriumcholat und 50 g Natriumdesoxycholat werden in 500 ml 0,9%iger Kochsalzlösung aufgenommen. Zu der so erhaltenen Lösung werden 0,5, 1,0, 1,5, 2,0 oder 4,0 g Cholesterin unter Rühren und unter Erwärmen in einem Wasserbad von ca. 50°C hinzugegeben. Nach dem Abkühlen auf Raumtemperatur wird mit 0,9%iger Natriumchloridlösung auf 1 l aufgefüllt.

Der erfindungsgemässe, so hergestellte Cholesterin-Standard zeigt bei der enzymatischen Bestimmung von Cholesterin mit Hilfe von Cholesterinoxidase aus Streptomyzes und 3,4-Dichlorphenol gleiche kinetische Eigenschaften wie Gesamtcholesterin enthaltende Proben, beispielsweise Kontrollseren, Humanseren usw.

*Beispiel 2*

50 g Natriumcholat und 50 g Natriumdesoxycholat sowie 1 g Natriumazid, 2,5 g Chloracetamid, 50 mmol Tris-Puffer, pH 8,0 ± 0,2 werden in 500 ml destilliertem Wasser aufgelöst. Die erhaltene Lösung wird über ein Membranfilter (0,2-0,4 µm) filtriert und danach auf ca. 50°C erwärmt. Es werden 0,5, 1,0, 1,5, 2,0 oder 4,0 g Cholesterin zugegeben und 10 bis 30 Minuten gerührt. Danach wird die Lösung erkalten gelassen und mit destilliertem Wasser auf 1 l aufgefüllt.

**Patentansprüche**

1. Wässrige Cholesterin-Standardlösung mit bestimmtem Gehalt an Cholesterin, dadurch gekennzeichnet, dass sie ein Detergensgemisch aus 10 bis 90% Cholsäure und 90-10% Desoxycholsäure bzw. aus geeigneten Salzen oder Derivaten dieser Säuren enthält.

2. Cholesterin-Standardlösung nach Anspruch 1, dadurch gekennzeichnet, dass das Detergensgemisch aus 40-60% Cholsäure und 60-40% Desoxycholsäure bzw. aus geeigneten Salzen oder Derivaten dieser Säuren besteht.

3. Cholesterin-Standardlösung nach Anspruch 1 oder 2, dadurch gekennzeichnet, dass als Detergens ein Gemisch von Natriumcholat und Natriumdesoxycholat zu gleichen Anteilen verwendet wird.

4. Cholesterin-Standardlösung nach einem der Ansprüche 1 bis 3, dadurch gekennzeichnet, dass das Detergensgemisch in einer Konzentration von 10 bis 300 g/l eingesetzt wird.

5. Cholesterin-Standardlösung nach einem der Ansprüche 1 bis 4, dadurch gekennzeichnet, dass sie zusätzlich 0,1-10 g/l eines oder mehrerer Konservierungsmittel und 10-100 mmol/l eines geeigneten, im pH-Bereich 7-9 wirksamen Puffers enthält.

6. Cholesterin-Standardlösung gemäss Anspruch 5, dadurch gekennzeichnet, dass sie

| 50-400 mg/dl | Cholesterin |
| 50 g/l | Natriumcholat |
| 50 g/l | Natriumdesoxycholat |
| 1,0 g/l | Natriumazid |
| 2,5 g/l | Chloracetamid |
| 50 mmol/l | Tris-Puffer, pH 8,0 ± 0,2 |

enthält.

7. Verfahren zur Herstellung einer wässrigen Cholesterin-Standardlösung gemäss einem der Ansprüche 1 bis 6, dadurch gekennzeichnet, dass man das Detergensgemisch aus Cholsäure und Desoxycholsäure bzw. aus geeigneten Salzen und Derivaten dieser Säuren in destilliertem Wasser oder 0,9%iger Natriumchlorid-Lösung auflöst, gegebenenfalls geeignete Konservierungsmittel und einen im pH-Bereich 7-9 wirksamen Puffer hinzugibt und in der so erhaltenen Lösung eine bestimmte, genau definierte Menge Cholesterin unter Rühren und Erwärmen auf 40-60°C auflöst.

## Claims

1. Aqueous cholesterol standard solution with a definite content of cholesterol, characterised in that it contains a detergent mixture of 10-90% of cholic acid and 90-10% of desoxycholic acid or of suitable salts or derivatives of these acids.

2. Cholesterol standard solution according to claim 1, characterised in that the detergent mixture consists of 40-60% cholic acid and 60-40% desoxycholic acid or of suitable salts or derivatives of these acids.

3. Cholesterol standard solution according to claim 1 or 2, characterised in that, as detergent, these is used a mixture of sodium cholate and sodium desoxycholate in equal proportions.

4. Cholesterol standard solution according to one of claims 1 to 3, characterised in that the detergent mixture is used in a concentration of 10-300 g/l.

5. Cholesterol standard solution according to one of claims 1 to 4, characterised in that it additionally contains 0.1-10 g/l of one or more preservation agents and 10-100 mmol/l of a suitable buffer effective in the pH range of 7-9.

6. Cholesterol standard solution according to claim 5, characterised in that it contains:

| 50-400 mg/dl | cholesterol |
| 50 g/l | sodium cholate |
| 50 g/l | sodiumdesoxycholate |
| 1,0 g/l | sodium azide |
| 2,5 g/l | chloroacetamide |
| 50 mmol/l | tris buffer, pH 8,0 ± 0,2. |

7. Process for the preparation of an aqueous cholesterol standard solution according to one of claims 1 to 6, characterised in that one dissolves the detergent mixture of cholic acid and desoxycholic acid or of suitable salts and derivatives of these acids in distilled water or 0.9% sodium chloride solution, optionally adds thereto suitable preservation agents and a buffer effective in the pH range of 7-9 and dissolves a definite, precisely defined amount of cholesterol in the so obtained solution, with stirring and warming to 40-60°C.

## Revendications

1. Solution étalon aqueuse de cholestérol ayant une teneur déterminée en cholesterol, caractérisée en ce qu'elle contient un mélange de détergents constitué de 10 à 90% d'acide cholique et de 90 à 10% d'acide désoxycholique ou de sels ou de dérivés appropriés de ces acides.

2. Solution étalon de cholestérol selon la revendication 1, caractérisée en ce que le mélange de détergents est constitué de 40 à 60% d'acide cholique et de 60 à 40% d'acide désoxycholique ou de sels ou de dérivés appropriés de ces acides.

3. Solution étalon de cholestérol selon la revendication 1 ou 2, caractérisée en ce que le détergent est constitué par un mélange de proportions égales de cholate de sodium et de désoxycholate de sodium.

4. Solution étalon de cholestérol selon l'une des revendications 1 à 3, caractérisée en ce que le mélange de détergents est mis en oeuvre en une concentration de 10 à 30 g/l.

5. Solution étalon de cholestérol selon l'une des revendications 1 à 4, caractérisée en ce qu'elle contient en outre de 0,1 à 10 g/l d'un ou de plusieurs agents de conservation et de 10 à 100 mmoles/l d'un tampon approprié actif entre pH 7 et pH 9.

6. Solution étalon de cholestérol selon la revendication 5, caractérisée en ce qu'elle contient:

| 50-400 mg/dl | de cholestérol |
| 50 g/l | de cholate de soidum |
| 50 g/l | de désoxycholate de sodium |
| 1,0 g/l | d'azaoture de sodium |
| 2,5 g/l | de chloracétamide |
| 50 mmoles/l | de tampon au tris, pH 8,0 ± 0,2. |

7. Procédé de préparation d'une solution étalon aqueuse de cholésterol selon l'une des revendications 1 à 6, caractérisé en ce qu'on dissout le mélange de détergents constitué d'acide cholique et d'acide désoxycholique ou de sels ou de dérivés appropriés de ces acides dans de l'eau distillée ou dans une solution de chlorure de sodium à 0,9%, on ajoute éventuellement des agents de conservation appropriés et un tampon actif entre pH 7 et pH 9 et on dissout dans la solution ainsi obtenue une quantité déterminée de cholésterol définie avec précision en agitant et en chauffant à une température entre 40 et 60°C.